Europäisches Patentamt

European Patent Office

Office européen des brevets

(11) Veröffentlichungsnummer: **0 288 829 B1**

(12) **EUROPÄISCHE PATENTSCHRIFT**

(45) Veröffentlichungstag der Patentschrift: **01.07.92**

(21) Anmeldenummer: **88105964.6**

(22) Anmeldetag: **14.04.88**

(51) Int. Cl.5: **C12N 1/20**, //A01N63/00, C12P21/00,(C12N1/20, C12R1:07)

(54) Verfahren zur Gewinnung von insektenpathogenen Proteinen und Mikroorganismen des Typs Bacillus thuringiensis.

(30) Priorität: **25.04.87 DE 3713946**

(43) Veröffentlichungstag der Anmeldung:
**02.11.88 Patentblatt 88/44**

(45) Bekanntmachung des Hinweises auf die Patenterteilung:
**01.07.92 Patentblatt 92/27**

(84) Benannte Vertragsstaaten:
**AT BE CH DE FR GB IT LI NL**

(56) Entgegenhaltungen:
**EP-A- 0 195 285**

**MICROBIOLOGICAL REVIEWS, Band 50, Nr. 1, März 1986, Seiten 1-24, American Society for Microbiology; A.I. ARONSON et al.: "Bacillus thuringiensis and related insect pathogens"**

**PROCEEDINGS OF THE NATIONAL ACADEMI-CAL SOCIETY, Band 79, November 1982, Seiten 6951-6955; J.M. GONZALEZ et al.: "Transfer of bacillus thuringiensis plasmids coding for delta-endotoxin among strains of B. thuringiensis and B. cereus"**

(73) Patentinhaber: **BASF Aktiengesellschaft Carl-Bosch-Strasse 38 W-6700 Ludwigshafen(DE)**

(72) Erfinder: **Krieg, Wolfgang, Dr. Saarstrasse 17 W-6721 Weingarten(DE)**
Erfinder: **Zaehner, Hans, Prof. Dr. Im Hopfengarten 13 W-7400 Tuebingen(DE)**
Erfinder: **Bernhard, Konrad, Dr. Unterdorfstrasse 44 W-7850 Loerrach-Hauingen(DE)**
Erfinder: **Schall, Dietmar, Dr. 551 Atwood Ct. Newtown, PA 18940(US)**

Rank Xerox (UK) Business Services

**Beschreibung**

Neben synthetischen und aus gewissen Pflanzen isolierten Insektiziden sind bakterielle Insektizide bekannt; es handelt sich um Proteine, die im Wildtyp und in verschiedenen Mutanten des seit 1915 bekannten Bacillus thuringiensis (B.t.) gebildet werden.

Bacillus Arten bilden bei ungünstigen Umweltbedingungen hitze- und austrocknungsresistente Überdauerungsstadien (Endosporen). Bacillus thuringiensis bildet aber während der Sporulation zusätzlich einen im Lichtmikroskop deutlich sichtbaren parasporalen Einschlußkörper, der aufgrund seines kristallähnlichen Aufbaus vereinfacht als Kristall bezeichnet wird. Diese Proteinkristalle sind für die Toxizität von B.t. verantwortlich (Angus 1954). Sie lösen sich nach oraler Aufnahme im alkalischen Milieu des Darms der Zielinsekten auf. Durch die Wirkung von Darmproteasen (Tojo et al. 1985) bzw. kristallassoziierten Proteasen (Turley et al. 1985) entstehen dabei noch nicht genau bekannte toxische Spaltprodukte (Delta-Endotoxine), die schließlich zur Lyse von Darmepithelzellen führen. Der molekulare Mechanismus der Delta-Endotoxinwirkung ist noch nicht genau bekannt.

Die heute übliche Klassifizierung der vielen isolierten B.t.-Stämme erfolgt durch Vergleich der Flagellen-Antigene (De Barjac und Bonnefoi 1973; De Barjac 1981). Danach lassen sich bis heute 22 H-Antigen Gruppen unterscheiden. Außerdem werden die Stämme auch aufgrund ihres Wirkungsspektrums in Pathotypen eingeteilt. Mehr als 95 % der bisher gefundenen B.t.-Isolate bilden rhombische Kristalle und sind gegen Schmetterlingslarven wirksam (Pathotyp A). Im Pathotyp B sind Varietäten mit Aktivität gegen Stechmückenlarven und rundlichen, unregelmäßigen Kristallen und im Pathotyp C Stämme mit Aktivität gegen Käferlarven und plättchenförmigen Kristallen zusammengefaßt. Für einige kristallbildende B.t.-Unterarten (z.B. tochigiensis, kumamotoensis) ist keine insektizide Wirkung bekannt (Pathotyp ND). Außerdem sind auch schmetterlingspathogene Stämme beschrieben worden, die eine geringe mosquitozide Aktivität besitzen. Diese Isolate bilden neben dem lepidopteren-spezifischen Kristall (Protein P1: Molekulargewicht ca. 130 000) noch einen kleinen ovalen Einschlußkörper (Protein P2: Molekulargewicht ca. 65 000), der eine schwache Wirkung gegen Schmetterlinge und Stechmücken besitzt (Iizuka und Yamamoto 1983).

Serotyp und Pathotyp sind nicht korrelierbar. So sind z.B. Pathotyp A, B und C Stämme mit denselben Flagellen-Antigenen (H8) isoliert worden.

In allen B.t.-Stämmen sind 2 bis 12 Plasmide mit Molekulargewichten zwischen 1,5 und >150 Md nachweisbar (Carlton und Gonzalenz 1985a). Curingversuche (Gonzalez et al. 1981), Kreuzungsversuche (Gonzalez et al. 1982) und Klonierungsversuche (Mc Linden et al. 1985) haben gezeigt, daß die Fähigkeit zur Kristallbildung in vielen Stämmen plasmiddeterminiert ist. Dabei können die beteiligten Gene auf einem oder mehreren Plasmiden (30-150 Md) liegen. Hybridisierungsversuche mit klonierten Kristallgenen ergaben eine große Ähnlichkeit der Delta-Endotoxin-Gene innerhalb der Pathotyp A Gruppe (Lereclus et al. 1982; Kronstadt et al. 1983). Durch Klonierung eines Delta-Endotoxin-Gens können weitere Kristallgene desselben Pathotyps daher sehr einfach durch Hybridisierung mit der spezifischen Probe gefunden werden. Auf diese Weise konnten z.B. mindestens 3 Delta-Endotoxin-Gene in B.t.kurstaki HD-1 nachgewiesen werden (Kronstad und Whiteley 1986).

Die Funktion der meisten Plasmide in B.t. ist unbekannt. Außer den Kristallproteingenen sind erst zwei weitere plasmidcodierte Eigenschaften beschrieben worden. Tam und Fitz-James (1986) konnten die Bildung von phagenartigen Partikeln in B.t.israelensis einem 68 Md Plasmid zuordnen. Diese Partikel werden auch während der Sporulation gebildet und sind neben dem Delta-Endotoxin-Kristall als weiterer kleiner Einschlußkörper sichtbar. Schließlich war von Ozawa und Iwahana (1986) die Kopplung der Kristallbildung und der β-Exotoxinbildung (Sebesta et al. 1981) an ein 62 Md-Plasmid in B.t.darmstadiensis nachweisbar.

B.t.-Stämme sind physiologisch nicht von Bacillus cereus unterscheidbar (Baumann et al. 1984). In B.cereus Stämmen, die aus Bodenproben isoliert wurden, konnten alle bisher bei B.t. charakterisierten H-Antigene gefunden werden (Ohba und Aizawa 1986). Der einzige Unterschied zwischen B.t. und B.cereus ist daher die Bildung des Delta-Endotoxin-Kristalls während der Sporulation. Diese Eigenschaft kann aber durch Plasmidcuring leicht verloren gehen. B.t. muß somit als Varietät von B.cereus angesehen werden.

Die Wirkungsspektren verschiedener Stämme sind auch innerhalb desselben Pathotyps sehr unterschiedlich (Krieg und Langenbruch 1981). Für eine optimale Insektenbekämpfung ist deshalb der Einsatz von verschiedenen B.t.-Stämmen sinnvoll. Da die Wachstumseigenschaften von verschiedenen B.t.-Varietäten sehr unterschiedlich sind, ist für jeden Stamm eine eigene Fermentationsoptimierung notwendig. Dies scheiterte bisher an den dafür erforderlichen hohen Kosten. Eine Möglichkeit, diese Situation zu verbessern, wäre die Verwendung eines Produktionsstammes, in den dann je nach Bedarf die genetische Information zur Bildung von verschiedenen Delta-Endotoxinen eingeführt wird. Der Transfer von Kristallplasmiden war bisher nur durch den von Gonzalez et al. (1982) entdeckten konjugationsähnlichen Mechanismus möglich.

Mit dieser Methode konnten Plasmide von schmetterlingsaktiven Stämmen mit hoher Rate (bis 80 %)) in cry⁻-Stämme, sogar in B.cereus übertragen werden. Die Übertragung von Plasmiden ist aber stark von den Kreuzungspartnern abhängig. Nur in bestimmten Kreuzungen war eine Übertragung der Kristallbildung nachweisbar.

Eine molekulargenetische Bearbeitung der Delta-Endotoxine wird dadurch erleichtert, daß die beteiligten Gene in der Regel auf Plasmiden lokalisiert sind. Es sind schon mehrere Kristallgene in E.coli oder B.subtilis kloniert und sequenziert worden (Schnepf und Whiteley 1981; Held et al. 1982; Klier et al. 1982; Shibano et al. 1985; Schnepf et al. 1985; Adang et al. 1985). Charakteristisch für die Delta-Endotoxin Produktion in B.t. ist die hohe Expression (bis 30 % der sporulierten Zelle). In E.coli bzw. B.subtilis ist die Expression mit klonierten Genen bedeutend schlechter. Dies ist z.B. durch unterschiedliche Regulationsmechanismen in diesen Organismen erklärbar. Um die Probleme auszuschließen, wäre die Untersuchung der Delta-Endotoxine direkt in B.thuringiensis oder B.cereus wünschenswert. Da keine geeignete Transformationsmethode ihn B.t. vorhanden war, konnten solche Versuche bisher nicht durchgeführt werden (Carlton und Gonzalez 1985b; Aronson et al 1986).

Aufgabe der Erfindung ist die Schaffung eines effizienten Transformationssystems, mit dem möglichst alle Bacillus thuringiensis und Bacillus cereus Stämme transformiert werden können, so daß neue Stämme entstehen, die die Fähigkeit zur Produktion von mehreren Toxinen im gleichen Organismus besitzen. Eine Möglichkeit, bei fehlender natürlicher Kompetenz eine Aufnahme von DNA zu induzieren, ist die Protoplastentransformation (Bibb et al. 1978). Diese Methode war die Grundlage des erfindungsgemäßen Transformationsverfahrens. Die Protoplastentransformation wurde aus der Protoplastenfusion (Schaeffer et al. 1976; Fodor und Alföldi 1976) entwickelt. Bei der Zellfusion wird durch das Verschmelzen der Zellmembranen eine Kombination der Zellinhalte beider Elternstämme erreicht, wobei eine Rekombination von Genen und eine Neuverteilung von Plasmiden eintreten kann.

Erfindungsgegenstand ist demnach ein Verfahren zur Gewinnung von Mikroorganismen des Typs Bacillus thuringiensis, die genetisch fixiert die Fähigkeit zur Bildung von Endotoxinen haben, durch Protoplastenfusion zweier bzw. mindestens zweier, unterschiedliche Toxine aufweisender Stämme, wobei in einem ersten Schritt die Zellwand abgebaut, in einem zweiten Schritt die Fusion induziert und schließlich die Regeneration der Zellwand wieder angeregt wird.

Erfindungsgemäß ist insbesondere ein neuer Stamm von Bacillus thuringiensis mit einer hohen Aktivität gegen Larven von Schadschmetterlingen und Käfern.

Der Stamm trägt die Bezeichnung Bacillus thuringiensis F 8-1 und ist unter der Nr. 4082 bei der Deutschen Sammlung für Mikroorganismen, D-3400 Göttingen, hinterlegt.

Um eine Protoplastierung der Zellen zu erreichen, muß zuerst die Zellwand abgebaut werden. Dies wird durch Inkubation der Zellen in einem osmotisch stabilisierten Puffer mit Lysozym erreicht. Eine Transformation bzw. Fusion wird durch hohe Konzentrationen an Polyethylenglykol induziert. Die Wirkung des Polyethylenglykols ist dabei nicht genau bekannt. Nach der eigentlichen Transformation oder Fusion muß von den Protoplasten wieder eine intakte Zellwand aufgebaut werden (Regeneration). Ein wichtiger Schritt ist die Selektion von transformierten bzw. fusionierten Klonen. Dazu gibt es zwei Möglichkeiten: Bei der indirekten Selektion werden die Protoplasten zuerst auf einem geeigneten Medium regeneriert. Anschließend erfolgt die Selektion, z.B. durch das Überstempeln auf ein Selektionsmedium. Vorteilhafter ist die direkte Selektion. Hier können durch die Wahl von geeigneten Bedingungen nur selektierte Kolonien auf dem Regenerationsmedium wachsen.

Die zur Zeit bekannten insektentoxischen B.t.-Isolate können aufgrund der Wirkungsspektren ihrer delta-Endotoxime in mehrere Pathotypen eingeteilt werden. Dem Pathotyp A ordnet man Stämme mit Wirkung gegen Lepidopteren zu, dem Pathotyp B solche mit Wirkung gegen Dipteren und dem Pathotyp C solche mit Wirkung gegen Coleopteren.

Tabelle 1

| Serovarietät | Name | Pathotyp | Stamm, z.B. |
|---|---|---|---|
| 1 | thuringiensis | A | HD-1, HD-20 |
| 3a 3b | kurstaki | A | HD-1, HD-73 |
| 5a 5b | galleriae | A | HD-29, HD-950 |
| 6 | entomocidus | A | HD-967 |
| 7 | aizawai | A | HD-135, HD-980 |
| 8a 8b | morrisoni (tenebrionis PG 14) | A, B, C | HD-12, BI 256-82 |
| 11 | kyushuensis | C | |
| 14 | israelensis | C | A60-, HD-567 |

Tabelle 1 gibt eine Übersicht mit ausgewählten Beispielen, Serotyp und Pathotyp sind nicht korrelierbar. So sind z.B. Pathotyp A, B und C-Stämme mit demselben Flagellen-Antigenen (H8) isoliert worden.

Die bisher kommerziell erhältlichen Präparate beruhen auf wenigen Pathotyp A-Stämmen zur Bekämpfung einiger Schmetterlingsarten, die im Gemüse- und Obstbau sowie im Forst schädlich werden, und auf einem Pathotyp B-Stamm zur Bekämpfung von Stechmücken- und Kriebelmückenlarven.

Insbesondere beim Einsatz in landwirtschaftlichen Kulturen können die bekannten Stämme bzw. Toxine nicht immer vollends überzeugen. Um für den Anwender eine praktikable Alternative zu bekannten, insbesondere synthetischen Wirkstoffen zu sein, müssen neue Stämme mit verbesserten Eigenschaften gefunden werden, d.h. höhere Toxizität gegen bestimmte Schaderreger, erweitertes Aktivitätsspektrum z.B. gegen viele Lepidopterenarten und/oder kombiniertes Spektrum z.B. gegen die Larven von Lepidopteren und Coleopteren.

Da jedes einzelne Isolat nur ein enges Wirkungsspektrum aufweist, sollten aus einer Vielzahl insektizider Spektren durch Kombination neue Stämme geschaffen werden können.

Eigenschaften der Kombinationsstämme müssen hinsichtlich Produktion und Anwendung denen einer bloßen Mischung zweier Einzelstämme überlegen sein.

In allen B.t.-Stämmen sind 2 bis 12 Plasmide mit Molekulargewichten zwischen 1,5 und >150 Md nachweisbar (Carlon, Gonzales, 1985 in Molecular Biology of Bacilli Academic Press, Curingversuche (Gonzales et al. 1981, Plasmid 5 351 ff, Kreuzungsversuche (Gonzales et al. 1982, Proc. Notl. Acad. Sci. USA 79, 6951 ff und Klonierungsversuche (Mc Linden et al. 1985, Appl. Environm. Microbiol. 50, 620 ff) haben gezeigt, daß die Fähigkeit zur Kristallbildung in vielen Stämmen plasmidcodiert ist. Dabei liegen die beteiligten Gene auf einem oder mehreren Plasmiden (30-150 Md).

Hybridisierungsversuche mit klonierten Kristallgenen ergaben große Ähnlichkeit der delta-Endotoxin-Gene innerhalb der Pathotyp A-Gruppe (Lereclus et al. 1982, Mol. Gen. Genetic. 186, 391 ff; Kronstad et al. 1983, J. Bacteriol. 154, 419 ff).

Durch Klorierung eines Gens können weitere Kristallgene desselben Pathotyps durch Hybridisierung mit der spezifischen Probe gefunden werden. Auf diese Weise wurden z.B. mindestens drei delta-Endotoxin-Gene in B.t. Var. kurstaki (HD-1) nachgewiesen (Kronstad, Whiteley 1986, Gene 43, 29 ff).

B.t.-Stämme sind physiologisch nicht von B. cereus unterscheidbar (Baumann et al. 1984). In B. cereus-Stämmen, die aus Bodenproben isoliert wurden, konnten die bisher bei B.t. charakterisierten H-Antigene gefunden werden (Ohba, Aizawa 1986, J. Basic. Microbiol. 26, 185 ff). Der einzige Unterschied zwischen B.t. und B.c. ist daher die Bildung des delta-Endotoxinkristalls während der Sporulation.

Ein Transfer von Plasmiden, isoliert aus B.t., ist in verschiedenen Arbeiten publiziert. In den meisten Fällen handelt es sich bei dem Wirt, in dem die Gene oder Genfragmente exprimiert werden, um E. coli (US Patent 4 448 885, EP 063039, EP Appln. 93 062, EP Appln. 186 379. Aber auch in B. megaterium und B. subtilis ist z.B. die Expression von Bti-Toxin möglich (EP 195 285).

Als weitere Arbeiten zu diesem Thema können herangezogen werden: Schnepp, Whitely 1981; Proc. Natl. Acad. Sci USA 78, 2893 ff, Kronstad et al. 1983, J. Bacteriol. 154, 419 ff, Held et al. 1982, Proc. Natl. Acad. Sci, USA 79. 6065 ff, Klier et al. 1982, EMBO Journal 1, 791 ff.

In E. coli und den anderen Wirten ist aber die Expression der klonierten Gene in der Regel schlecht, im Gegensatz zu der im originalen Wirt B.t., für den eine hohe delta-Endotoxin-Produktion von bis zu 30 % der sporulierten Zelle charakteristisch ist.

Im folgenden wird nun ein Protoplasten-Flüssigsystem angegeben, mit dem es möglich ist, genetische Informationen bidirektional zu übertragen. Das primäre Fusionsprodukt ist eine diploide Zelle, in der die Gesamt-DNA aus beiden Elternstämmen vorhanden ist. Nach Segregation in haploide stabile Zellen liegen Mischungen der Plasmide aus beiden Eltern vor. Man findet in einer hohen Rate Klone, bei denen die Fähigkeit zur Kristallbildung von einem Elter in den anderen übertragen ist.

Voraussetzung für die Fusion ist die Protoplastierung der Zellen (Schaeffer et al. 1976, Proc. Natl. Acad. Sci. USA 79, 2151 ff, Foder, Alföldi 1976, Proc. Natl. Acad. Sci. USA 73 2142 ff). Einen Abbau der Zellwand erreicht man durch Inkubation der Zellen in einem osmotisch stabilisierten Puffer mit Lysozym.

Die Fusion wird durch hohe Konzentrationen an Polyethylenglykol (PEG) induziert. Nach vollzogener Fusion muß von den Protoplasten wieder eine intakte Zellwand aufgebaut werden (Regeneration).

Ein wichtiger Schritt ist die Selektion von fusionierten Klonen; es gibt zwei Möglichkeiten: bei der indirekten Selektion werden die Protoplasten zuerst auf einem geeigneten Medium regeneriert, anschließend erfolgt die Selektion, z.B. durch Überstempeln auf ein Selektionsmedium, vorteilhafter ist die direkte Selektion; hier können durch Wahl von geeigneten Bedingungen nur selektierte Kolonien auf dem Regenerationsmedium wachsen.

Zur Vereinfachung der Selektion wurden auxotrophe und apathogene $cry^-$-Mutanten konstruiert sowie mittels Transformation Antibiotika-Markern enthaltende Phaenotypen.

Prinzipiell lassen sich mit der beschriebenen Technik alle denkbaren delta-Endotoxin-Kombinationen konstruieren, was biologisch zu veränderten, breiteren, aktiveren Eigenschaften führen kann.

Folgende Stämme können z.B. als Donor fungieren:

```
No.

    1    Bacillus thuringiensis    var. thuringiensis   z.B. HD 2,  HD 20

    2                              var. finitimus             -

 3a  3b                           var. kurstaki        z.B. HD 1


    3a                            var. alesti


 4a  4b                           var. sotto                HD-772

 4a  4b                           var. dendrolimus

 4a   4b                          var. kenyae

 5a   5b                          var. galleriae            HD 29,  HD 950


    6                             var. subtoxicus

                                  var. entomocidus          HD 967

    7                             var. aizawai             HD 980, HD 135

 8a  8b                           var. morrisoni            HD 12

                                  var. tenebrionis          Bi-256-82

                                  PG 14

    9                             var. tolworthi

   10                             var. darmstadiensis

11a 11b                           var. toumanoffi

11a 11c                           var. kyushuensis


   12                             var. thompsoni

   13                             var. pakistani

   14                             var. israelensis        A60, HD 567, HD 968

   15                             var. dakota

   16                             var. indiana

   17                             var. tohokuensis

   18                             var. kumamotoensis

   19                             var. yunnanensis

   21                             var. colmeri


    -                             var. wuhanensis


Bacillus sphaericus ATCC 29 203
Bacillus popilliae  ATCC 14 706
```

Diese Stämme sind allgemein zugänglich.

Die vorgenannten Stämme sind auch als Rezipient geeignet; eingesetzt werden können aber z.B. auch:

| | |
|---|---|
| Bacillus subtilis | DS 11 402 |
| Bacillus cereus | ATCC 21 281, DSM 31 351 |
| E.coli | |
| Bacillus megaterium | |

Eine zusätzliche Möglichkeit, die ökotoxikologische Belastung des zu behandelten Biotopes auf ein Minimum zu reduzieren, ist die Züchtung von sporenlosen Mutanten dieser durch Protoplastenfusion gewonnenen Stämme. Beispiele zur Sporenlosigkeit sind z.B. in EP-A-59 460 oder EP-D-178 151 beschrieben.

Beispiel 1

Die Tabelle beschreibt eine Auswahl von konstruierten Stämmen, bei denen verschiedene Eigenschaften mittels Protoplastenfusion übertragen wurden. Die Darstellung beschränkt sich auf Stämme mit Kristallbildung.

| Stamm | Biol. Wirksamkeit | |
|---|---|---|
| | Pathotyp A | Pathotyp C |
| HD-2 cry⁺thur (wildtyp) | + | - |
| HD-2 cry⁻ | - | - |
| HD-2 cry⁻ x HD-2 cry⁺thur. | + | - |
| HD-2 cry⁻ x HD-73 cry⁺ kurstaki | + | - |
| Bl 256-82 cry⁺ ten (Wildtyp) | - | + |
| HD-2 cry⁻ Bl 256-82 cry⁺ten (F32-13) | - | + |
| F32-13 x HD-2 cry⁺thur. (F8-1) | + | + |
| HD-2 cry⁻ x HD-8 cry⁺gal. (F19-.30) | + | - |
| B.cereus HM7-1 | - | - |
| B.cereus HM7-1x HD-8 cry⁺gal. (F12-1) | + | - |
| B.cereus HM7-1 HD-2 cry⁺thur. (F4-3) | + | - |
| B.cereus HM7-1 HD-73 cry⁺kurstaki (F10) | + | - |

Beispiel 2

Die Tabelle gibt eine Übersicht zu Empfindlichkeit von B.t.-Stämmen hinsichtlich der Protoplastierung in verschiedenen Medien STML, MML und SMMP M/L.

Beispiel

| | STML | MML | SMMP M/L |
|---|---|---|---|
| BI 256-82 | ++ | ++A | +A |
| A 60 | ++ | - | - |
| PG 14 | ++ | +A | -/+ |
| HD 8 PC 194 | ++ | ++A | - |
| HD 70 30 | ++ | ++ | + |
| HD 1 | ++ | ++ | -/+ |
| HD 2 D 26 | ++ | + | ++ |
| B.C. DSM 31 | ++ | ++ | -/+ |

A = Aggregation

3

Tabelle gibt ein Beispiel für eine homologe Fusion zwischen auxotrophen Mutanten des Stammes HD 2.

Tabelle: Homologe Fusion zwischen auxotropen Mutanten des Stammes HD 2 (var. thuringiensis)

7

| Eltern: | |
|---|---|
| "Rezipient" D6-4 | met$^-$, arg$^+$, Sm$^r$, Tc$^s$, crys$^-$ |
| "Donor" D12 (pBC 16) | met$^+$, arg$^-$, Sm$^s$, Tc$^r$, crys$^+$ |

Fusionsraten:    2,0 % Tc$^r$Sm$^r$ Kolonien 1,2 % prototrophe Kolonien

| Analyse der Tc$^r$Sm$^r$ Kolonien | | | |
|---|---|---|---|
| met | arg | crys | prozentualer Anteil |
| - | + | + | 25,6 |
| - | + | - | 3,6 |
| + | + | + | 48,8 |
| + | + | - | 1,2 |
| - | - | + | 9,8 |
| - | - | - | 1,2 |
| + | - | + | 9,8 |

Beispiel 4

Im folgenden wird der Arbeitsablauf für eine Protoplastenfusion kurz skizziert:

1) Ernte der Zellen von 5 ml Kultur (Autolysestadium oder Übernachkultur)

2) Zellen in 1 ml Protoplastierungspuffer resuspendieren

3) 2 h bei 28°C inkubieren

4) Protoplasten 2 × in SMMP-Medium waschen

5) Protoplasten in 0,5 ml SMMP-Medium resuspendieren

6) Fusionsansatz herstellen:

0,2 ml Protoplasten "Donor" + 0,2 ml Protoplasten "Rezipient" 0,3 ml 50 %iges PEG in SMM-Puffer

7) 5 min bei Raumtemperatur inkubieren

8) 5 ml SMMP-Medium zugeben und Protoplasten abzentrifugieren

9) Protoplasten in 1,5 ml SMMP-Medium resuspendieren

10) Protoplasten 5 h bei 28°C inkubieren

11) Verdünnungsreihe in SMM-Puffer ansetzen und Aliquots auf Regenerationsagar ausplattieren.

Beispiel 5

Tabelle gibt ein Beispiel für eine Fusion zwischen heterologen Stämmen, bei der die kristallbildende Eigenschaft übertragen wird.

Tabelle: Fusion zwischen heterologen Stämmen

| Fusion F6 | | |
|---|---|---|
| "Donor": | HD 73-30 (pBC 16) | met$^+$, arg$^-$, St$^s$, Tc$^r$, crys$^+$ |
| "Rezipient: | HD 2 D6-4 | met$^-$, arg$^+$, St$^r$, Tc$^s$, crys$^-$ |

Fusionsrate: 0,5 % St$^r$, Tc$^r$ Kolonien
St$^r$, Tc$^r$ Kolonien pro Platte: 10

Beispiel 6

Tabelle gibt ein Beispiel für eine Fusion, bei der zwei HD-2 Mutanten mit unterschiedlicher Kristallbildung kombiniert wurden

| "Donor": | F 32-19 | met$^-$, pur$^+$, cry$^+$ten |
|---|---|---|
| "Rezipient: | HD-2 D 10 | met$^+$, pur$^-$, cry$^+$thur. |

Fusionsrate: 0,7 %

Prototrophe Kolonien: >100/Platte

| Mikroskopische Überprüfung von Kolonien (76) | |
|---|---|
| Pathotyp A-Kristall | 26,3 % |
| Pathotyp C-Kristall | 7,9 % |
| A + C-Kristalle | 65,8 % |

Beispiel 7

Tabelle gibt ein Beispiel zur Fusion von B.t. Protoplasten mit B.c.-Protoplasten

Fusion F12

| "Donor" | B. thuringiensis HD 8 K4-3 (pBC 16) |
|---|---|
| "Rezipient" | B. cereus UM 7-1 (pCM 194)<br>his$^-$, nic$^+$, Sm$^r$, Tc$^s$, Cm$^r$, crys$^+$ |

| Analyse der Tc$^r$Sm$^r$ Kolonien | |
|---|---|
| his$^-$, nic$^+$, Cm$^r$, crys$^+$ | 4 |
| his$^-$, nic$^+$, Cm$^s$, crys$^+$ | 5 |
| his$^-$, nic$^+$, Cm$^s$, crys$^-$ | 2 |
| his$^-$, nic$^+$, Cm$^r$, crys$^-$ | 2 |
| | 13 |

Anwendungsbeispiel

In Tabelle ist die biologische Wirkung verschiedener fusionierter Stämme zusammengestellt.

Die Experimente wurden wie folgt durchgeführt:

a) Plutella maculipennis (Kohlmotte)

Fraß- und Kontaktversuch

Junge Kohlrabiblätter wurden 3 sek in die wäßrige Aufbereitung der Prüfsubstanz getaucht und auf einen mit 0,5 ml Wasser angefeuchteten Rundfilter in einen Plastikbecher gelegt. Daraufhin wird das Blatt mit 10 Larven im 2.-3. Stadium besetzt.

Die Beurteilung erfolgt auf Fraß und Mortalität 3 Tage nach Versuchsbeginn.

b) Spodoptera littoralis (Ägypt. Baumwolleule)

Zuchtversuch auf künstlichem Nährmedium

Die Zucht erfolgt in 100 ml Plastikbechern auf ca. 50 ml des Standard-Nährmediums, dem im flüssigen Zustand der Wirkstoff sorgfältig untergemischt wurde. Je Konzentration werden 10 Becher mit einer Larve (L3) von 10-12 mm Länge angesetzt. Die Beobachtung erstreckt sich in der Regel bis zum Schlüpfen der Falter.

c) Aedes aegypti (Gelbfiebermücke)

Kontakt- und Fraßversuch

250 ml Kunststoffbecher werden mit 200 ml Leitungswasser von 23°C gefüllt und mit 20 Aedeslarven (L2) besetzt. Daraufhin gibt man die Prüfsubstanz in das Gefäß und bestimmt nach 24 Stunden die Mortalität.

d) Leptinotarsa decemlineata (Kartoffelkäfer)

Fraß- und Kontaktversuch

Stanzstücke aus jungen Kartoffelblättern werden 3 sec in die wäßrige Aufbereitung der Prüfsubstanz getaucht und danach in Palettenfächer (1 Stanzstück/Fach) plaziert; pro Fach wird dann eine vorher ausgewogene Kartoffelkäferlarve (L3) eingesetzt; die Anzahl an Wiederholungen beträgt n = 10. Die Auswertung erfolgt 3 DAT auf Fraß, Gewichtsänderung der Larven (mg) und Mortalität.

Tabelle

| | Plutella % Mort. bei 100 ppm | Spodoptera % Mort. bei 1000 ppm | Aedes % Mort. bei 100 ppm | Leptinotarsa $\Delta$Gewicht (mg) bei 0,1 % |
|---|---|---|---|---|
| HD-8 K4-3 | 75 | 100 | 0 | +202 erfgem. |
| B.c. D1 | 0 | 20 | 0 | +194 |
| F12-1 | 70 | 100 | 0 | +202 |
| F4-3 | 65 | 60 | 0 | +189 |
| BI 256-82 | 0 | 0 | 0 | - 6 |
| F8-1 | 85 | 60 | 0 | + 2 erfgem. |
| Kontrolle (unbehandelt) | 10 | 0 | 0 | +251 |

HD-8 K4-3 = B.t. galleria + pBC 16 + pc 194 (Pathotyp A)

B.c. D1_ = B.cereus his$^+$ str$^r$

F12-1 = B.cereus fusioniert mit HD8 cry$^+$gal (Pathotyp A)

F4-3 = B.cereus fusioniert mit HD-2 cry$^+$thur (Pathotyp A)

BI 256-82 = B.t. tenebrionis (Pathotyp C)

F8-1 = F32-13 cry$^+$ten fusioniert mit HD-2 D10 cry$^+$ thur.

(Pathotyp A+C)

Angaben zu den verwendeten Nährlösungen

Beispiel 1

Rezepturen für Protoplastierungspuffer sind z.B. folgende:

1) STM: 0,5 M Saccharose, 30 mM Tris, 5 mM $MgCl_2$ × $6H_2O$ pH 8,0

2) STML: STM mit 0,5 mg/ml Lysozym

3) SMM: 0,5 M Saccharose, 20 mM $Na_2$-maleat, 20 mM $MgCl_2$ pH 6,50

4) MM: 20 mM $Na_2$-maleat, 20 mM $MgCl_2$; pH 6,5

5) MML: MM mit 5 mg/ml Lysozym

6) SMMP: Gleiche Volumen an 2×SSM und 4×Antibiotic Medium 3 wurden nach dem Autoklavieren zusammengegeben.

7) SMMP M/L: SMMP mit 1000 U/ml Mutanolysin und 1 mg/ml Lysozym

Beispiel 2

Die Zusammensetzung eines Mediums zur Regeneration von Protoplasten ist z.B. folgende:

| Caseinpepton | 10 g |
|---|---|
| Hefeextrakt | 5 g |
| Glucose | 5 g |
| NaCl | 2 g |
| Na-citrat | 2 g |
| $MgCl_2 \times 6H_2O$ | 0,5 g |
| $CaCl_2 \times 2H_2O$ | 0,4 g |
| Saccharose | 340 g |
| pH 7,0 | |
| Gelatine | 25 g |
| Stärke | 15 g |
| Agar | 25 g |

RC1:     R mit 1 $\mu$g/ml Chloramphenicol

RC2:     R mit 2 $\mu$g/ml Chloramphenicol

RTC1:    R mit 15 $\mu$g/ml Tetracyclin und 1 $\mu$g/ml Chloramphenicol.

Beispiel 3

| Sojamehl entfettet | 10,0 g/l |
|---|---|
| Kartoffelstärke | 5,0 g/l |
| Hefeautolysat | 2,0 g/l |
| $K_2HPO_4$, wasserfrei | 1,0 g/l |
| $MgSO_4 \times 7\,H_2O$ | 0,3 g/l |
| $CaCl_2 \times 6\,H_2O$ | 0,08 g/l |
| $MnCl_2 \times 4\,H_2O$ | 0,05 g/l |
| CuCl | 0,005 g/l |
| $ZnCl_2$ | 0,005 g/l |
| $FeCl_3$ | 0,005 g/l |

Zur Anwendung als Insektizid wird das erfindungsgemäß erhaltene insektizid wirkende Präparat bzw. Toxin in an sich bekannter Weise mit üblichen Additiven (Trägerstoffe, Haftmittel, Netzmittel etc.) versetzt und in eine geeignete Anwendungsform übergeführt. Das so formulierte Insektizid kann in Form eines Spritzpulvers, einer Suspension, als Granulat o.ä. eingesetzt werden.

**Patentansprüche**

1.    Bacillus thuringiensis DSM 4082 und dessen insektenpathogene Proteine synthetisierenden Mutanten.

2.    Bacillus thuringiensis DSM 4082.

3.    Verfahren zur Gewinnung von Mikroorganismen des Typs Bacillus thuringiensis, die genetisch fixiert die Fähigkeit zur Bildung von Endotoxinen haben, durch Protoplastenfusion zweier bzw. mindestens zweier, unterschiedliche Toxine aufweisender Stämme, dadurch gekennzeichnet, daß man in einem ersten Schritt die Zellwand abbaut, in einem zweiten Schritt die Fusion induziert und schließlich die Regeneration der Zellwand wieder anregt.

**Claims**

1.    Bacillus thuringiensis DSM 4082 and its mutants which synthesize proteins pathogenic to insects.

2.    Bacillus thuringiensis DSM 4082.

3.    A process for obtaining microorganisms of the Bacillus thuringiensis type, which are genetically fixed and have the ability to form endotoxins, by protoplast fusion of two or more strains having different toxins, wherein the cell wall is degraded in a first step, fusion is induced in a second step and finally regeneration of the cell wall is induced.

**Revendications**

1. Bacillus thuringiensis DSM 4082 et ses mutants synthétisant des protéines pathogènes pour les insectes.

2. Bacillus thuringiensis DSM 4082.

3. Procédé d'obtention de microorganismes du type Bacillus thuringiensis qui, fixés génétiquement, ont la capacité de former des endotoxines par fusion de protoplastes de souches présentant deux, ou aux moins deux, toxines différentes caractérisé par le fait que, dans un premier temps, on détruit la paroi cellulaire, dans un deuxième temps, on induit la fusion et, enfin, on réanime la régénération de la paroi cellulaire.